# EUROPÄISCHE PATENTSCHRIFT

(11) **EP 1 115 682 B1**
(45) Veröffentlichungstag und Bekanntmachung des Hinweises auf die Patenterteilung: **26.11.2003**
(21) Anmeldenummer: 99969405.2
(22) Anmeldetag: 21.09.1999
(51) Int. Cl.: C07C 29/80, C07C 29/10, C07C 31/20

(54) **VERFAHREN ZUR HERSTELLUNG VON HOCHREINEM MONOETHYLENGLYKOL**
METHOD FOR PRODUCING HIGHLY PURE MONOETHYLENE GLYCOL
PROCEDE DE PRODUCTION DE MONOETHYLENE GLYCOL DE HAUTE PURETE

(30) Priorität: 23.09.1998 DE 19843697
(43) Veröffentlichungstag der Anmeldung: 18.07.2001
(73) Patentinhaber: BASF AKTIENGESELLSCHAFT, 67056 Ludwigshafen (DE)
(72) Erfinder: THEIS, Gerhard, D-67133 Maxdorf (DE); ADRIAN, Till, D-67240 Bobenheim-Roxheim (DE); BESSLING, Bernd, D-67269 Grünstadt (DE); HASSE, Hans, D-67661 Kaiserslautern (DE); VANSANT, Frans, B-2920 Kalmthout (BE)
(74) Vertreter: Isenbruck, Günter, Dr.
(86) Internationale Anmeldenummer: EP9906968
(87) Internationale Veröffentlichungsnummer: WO00017141

(56) Entgegenhaltungen:
- EP-A- 0 032 665
- DE-A- 2 364 151
- WEBER, T ET AL.: "Ullmanns Encyclopädie der technischen Chemie, 4. Auflage, Band 8" 1974 , VERLAG CHEMIE , WEINHEIM/BERGSTR. XP002127152 Seite 200 -Seite 210

## Beschreibung

Die Erfindung betrifft ein Verfahren zur Herstellung von hochreinem Monoethylenglykol.

Monoethylenglykol wird großtechnisch durch Hydrolyse von Ethylenoxid, Entwässerung und Reindestillation hergestellt. Zur Verbesserung der Selektivität der Ethylenoxid-(im folgenden abgekürzt als EO bezeichnet)-Hydrolyse wird der Hydrolysereaktor mit einem großen Wasserüberschuß (Gewichtsverhältnis Wasser:EO=4:1 bis 15:1) betrieben. Dadurch kann der Anteil an höheren Glykolen, insbesondere an Diethylenglykol, Triethylenglykol usw. zurückgedrängt werden. Der Hydrolysereaktor wird üblicherweise bei Temperaturen von 120°C bis 250°C und Drücken von 30 - 40 bar betrieben. Das Hydrolyseprodukt wird zunächst entwässert, auf einen Restwassergehalt von 100 - 200 ppm und anschließend in die verschiedenen Glykole in reiner Form aufgetrennt.

Die Entwässerung erfolgt in der Regel in einer Kaskade von druckgestaffelten Kolonnen, mit abnehmendem Druck. Aus Gründen der Wärmeintegration wird in der Regel nur der Sumpfverdampfer der ersten Druckkolonne mit Fremddampf beheizt, alle weiteren Druckkolonnen dagegen mit den Brüden der jeweils voranstehenden Kolonne. Der Zulauf wird jeweils im Sumpf der Kolonnen unterhalb des ersten Bodens aufgegeben, da zur Trennung von Wasser und Glykolen kein Abtriebsteil notwendig ist. Je nach Wassergehalt des Hydrolysereaktoraustrags und des Druck/Temperaturniveaus des im Sumpfverdampfer der ersten Kolonne eingesetzten Fremddampfs besteht die Druckentwässerungskaskade aus 2 bis 7 Kolonnen. An die Druckentwässerung schließt sich eine Vakuumentwässerung an, die in der Regel in einer Kolonne mit Abtriebsteil erfolgt. Das bei der Entwässerung anfallende Wasser wird vor den Hydrolysereaktor zurückgeführt. Die entwässerte, Glykolmischung wird in mehreren Kolonnen in die Reinstoffe zerlegt. Die Produkte Monoethylenglykol, Di- und Triethylenglykol werden jeweils als Kopfprodukt abgezogen, alle weiteren höheren Glykole fallen in Form eines Gemischs unter der Bezeichnung Polyethylenglykole als Sumpfprodukt der letzten Kolonne an.

Herkömmliche Glykolanlagen besitzen neben den Produktströmen üblicherweise nur einen einzigen weiteren Auslaß, den sogenannten Acetaldehydpurge am Sumpfverdampfer der zweiten Druckentwässerungskolonne. Dort wird der unkondensierte Anteil der zur Beheizung eingesetzten Brüden der ersten Kolonne ausgeschleust. Somit können Nebenkomponenten, die entweder mit dem Wasser/EO-Strom in die Glykolanlage eingetragen werden oder infolge von Nebenreaktionen in der Glykolanlage gebildet werden, nur über den Acetaldehydpurge oder über die Produktströme aus der Anlage ausgeschleust werden. Letzteres beeinträchtigt die Produktqualität und ist somit unerwünscht.

Bislang wurden Glykolanlagen nur bezüglich ihrer Hauptaufgaben optimiert, insbesondere bezüglich der Energie- und Investitionskostenreduzierung bei der Entwässerung und Reindestillation. In jüngster Zeit werden zunehmend höhere Anforderungen an die Produktqualität von Monoethylenglykol gestellt, insbesondere bezüglich des Gehalts an Nebenkomponenten. Es gibt zwei Monoethylenglykol-Produktqualitäten: Technical Grade (Antifreeze Grade) mit geringeren Anforderungen an die Reinheit, für den Einsatz als Kühlmittel und Fiber Grade, mit strengen Anforderungen, zum Einsatz u.a. in der Kunstfaserproduktion. Die Spezifikationen für Fiber Grade betragen kundenabhängig für freie Aldehyde, berechnet als Acetaldehyd nach der spektrophotometrischen Bestimmungsmethode als blauer MBTH-Komplex 7 bis 20 ppm und Mindest-UV-Transmission bei 220 nm 76%-80% und bei 275 nm 90% - 95%. Zum Meßwert für die freien Aldehyde tragen insbesondere Formaldehyd, Acetaldehyd und Glykolaldehyd bei. Die UV-aktiven Substanzen, sogenannte UV-Spoiler, sind weitgehend unbekannt und schon in Konzentrationen von weniger als 1 ppm spezifikationsschädlich. Beispiele sind Acrolein und Crotonaldehyd.

Die DE-A 23 64 151 beschreibt ein Verfahren zur destillativen Gewinnung von Monoethylenglykol aus dem Hydrolyseprodukt von Ethylenoxid, umfassend eine Reihe von Druckentwässerungsschritten, gefolgt von einer Vakuumentwässerung und einer Reindestillation. Bei der Vakuumentwässerung wird Wasserdampf als Kopfstrom mit einem Gehalt von 0,5 Gew.-% an Monoethylenglykol abgezogen, mit einem Kopfstrom aus der letzten Druckentwässerungskolonne vereinigt, teilweise ausgeschleust, und im übrigen in das Verfahren zurückgeführt. Dadurch können sich Verunreinigungen in der Anlage aufpegeln und es können darüber hinaus durch Reaktion der hochreaktiven Verunreinigungen untereinander und mit dem Reaktionsgemisch zusätzliche schädliche Nebenprodukte entstehen. Dadurch entstehen höhersiedende Verunreinigungen, die destillativ sehr schwer oder überhaupt nicht vom Monoethylenglykol abtrennbar sind. Das nach diesem Verfahren gewonnene Monoethylenglykol entspricht daher nicht den strengen Spezifikationen zum Einsatz in der Kunstfaserproduktion, insbesondere im Hinblick auf die UV-Transmissionswerte.

Die JP-A-60,089,439 beschreibt ein Verfahren zur Reinigung von Glykol durch Vakuumdestillation unter Zufuhr von Inertgas. Mit dem Stickstoffstrom wird ein Teil der Nebenkomponenten ausgestrippt und ein hochreines Glykol, das für die Faserherstellung geeignet ist, erhalten. Das Verfahren hat jedoch den Nachteil, daß zur effektiven Ausschleusung von Nebenkomponenten große Mengen an Stickstoff benötigt werden. Dies führt zu unerwünschten Produktverlusten im Abgas und zu einer unzulässig starken fluiddynamischen Belastung der Destillationskolonne. Monoethylenglykol notwendig sind.

Es ist Aufgabe der Erfindung, ein einfaches destillatives Verfahren zur Gewinnung von hochreinem Monoethylenglykol zur Verfügung zu stellen, ohne die Verwendung von Zusatzstoffen oder von speziellen Werkstoffen. Die Ausschleusung der spezifikationsschädlichen Nebenkomponenten soll in überwiegend wäßrigen Abfallströmen, mit Glykolgehalten von maximal 1 Gew.-% erfolgen, wobei die Nebenkomponenten in den Abfallströmen um den Faktor 10 - 100 aufkonzentriert werden sollen, da ansonsten zuviel Abwasser anfällt.

Die Lösung besteht in einem Verfahren zur destillativen Gewinnung von hochreinem Monoethylenglykol aus dem Hydrolyseprodukt von Ethylenoxid durch Druckentwässerung, bevorzugt in einer Kaskade, Vakuumentwässerung und anschließender Reindestillation, wonach bei der Vakuumentwässerung ein wässriger Strom abgezogen wird, der Monoethylenglykol in einer Konzentration unterhalb von 1 Gew.-%, bevorzugt unterhalb von 0,1 Gew.-%, Mittelsieder und Leichtsieder enthält und der, gegebenenfalls nach weiterer Aufarbeitung, ausgeschleust wird, und wobei die Vakuumentwässerung in einer Vakuumentwässerungskolonne stattfindet und der wässrige Strom als Seitenstrom aus der Vakuumentwässerungskolonne abgezogen wird, oder wobei die Vakuumentwässerung in zwei Vakuumentwässerungskolonnen stattfindet und der wässrige Strom als Kopfstrom der zweiten Vakuumentwässerungskolonne abgezogen wird.

Besonders bevorzugt ist ein Verfahren, wonach zusätzlich zur obengenannten Lösung, die Druckentwässerung in einer Entwässerungskolonne mit Abtriebsteil mit mindestens einer Trennstufe, bevorzugt 2-10 Trennstufen, besonders bevorzugt mit 3-6 Stufen, stattfindet und wonach ein Teil des Kopfstroms der Entwässerungskolonne(n) mit Abtriebsteil ausgeschleust wird.

Es wurde gefunden, daß die spezifikationsschädlichen Nebenkomponenten an bestimmten Stellen im Verfahren besonders effektiv ausgeschleust werden können. Das Erkennen dieser Stellen im Verfahren ist nicht trivial, da das Verhalten der Nebenkomponenten aufgrund der komplexen Phasengleichgewichte bislang nicht ausreichend gut beurteilt werden konnte. Daher wurde in berkömmlichen großtechnischen Verfahren nur ein sehr grober Auslaß für extrem leichtsiedende Nebenkomponenten geschaffen, der sogenannte Acetaldehydpurge am Sumpfverdampfer der zweiten Druckentwässerungskolonne. Dieser Auslaß ist nicht optimiert, da das Verhalten der Nebenkomponenten weitgehend unbekannt war bzw. bei der Verfahrensgestaltung nicht berücksichtigt wurde.

Die Komponenten werden bezüglich ihrer Siedelage vorliegend in drei Klassen eingeteilt:
1. Leichtsieder, mit einer Flüchtigkeit größer der von Wasser (insbesondere Acetaldehyd, Formaldehyd in reinem Wasser, Acrolein),
2. Mittelsieder, mit einer Flüchtigkeit zwischen der von Wasser und Monoethylenglykol (insbesondere Formaldehyd in glykolhaltigen wässrigen Lösungen, Formaldehyd in wasserfreiem Monoethylenglykol, Glykolaldehyd, Crotonaldehyd) und
3. Schwersieder, mit einer geringeren Flüchtigkeit als Monoethylenglykol (insbesondere höhermolekulare Aldehyde, UV-Spoiler).

Erfindungsgemäß wird die Vakuumentwässerung so gefahren, daß ein wässriger Strom abgezogen wird, der weniger als 1 Gew.-% Monoethylenglykol, Mittelsieder und Leichtsieder enthält, und der, ggf. nach weiterer Aufarbeitung, ausgeschleust wird.

Die Vakuumentwässerung kann in einer Vakuumentwässerungskalonne stattfinden, wobei dann ein wässriger Strom, der Mittelsieder und Leichtsieder enthält, als Seitenstrom abgezogen wird. Der Vakuumentwässerungskolonne wird ein Strom mit 1-99 Gew.-%, bevorzugt 50-90 Gew.-% Monoethylenglykol und 1-99 Gew.-%, bevorzugt 50-10 Gew.-% Wasser zugeführt, der spezifikationsschädliche Nebenkomponenten im Bereich von 1 ppm bis 5 %, bevorzugt im Bereich von 1 ppm bis 1 % , besonders bevorzugt im Bereich von 1 ppm bis 1000 ppm, enthält. Die Vakuumentwässerungskolonne wird dann in der Weise gefahren, daß ein Kopfprodukt überwiegend aus Wasser, mit einem Monoethylenglykolgehalt von unterhalb 5 Gew.-%, bevorzugt unterhalb 1 Gew.-%, bevorzugt unterhalb 1000 ppm und ein Sumpfprodukt aus überwiegend Glykol, mit einem Wassergehalt von unter 5 Gew.-%, bevorzugt unter 1 Gew.-%, besonders bevorzugt unter 1000 ppm, abgezogen wird. Der Vakuumentwässerungskolonne wird ein Seitenstrom entnommen, der weitgehend frei von Monoethylenglykol ist, d.h. mit einem Monoethylenglykolgehalt von unter 5 Gew.-%, bevorzugt unter 1 Gew.-%, besonders bevorzugt unter 1000 ppm, der an spezifikationsschädlichen Nebenkomponenten, insbesondere Mittelsiedern sowie auch Leichtsiedern, angereichert ist. Die Entwässerungskolonne wird bei einer Sumpftemperatur von höchsten 220°C, bevorzugt 120°C bis 200°C, besonders bevorzugt von 160°C bis 180°C, betrieben.

Der Zulauf zur Vakuumentwässerungskolonne ist in der Regel der Sumpfaustrag der Druckentwässerungskolonne bzw. der letzten Kolonne der Druckentwässerungskaskade. In Einzelfällen ist es jedoch auch möglich, direkt den Austrag eines EO-Hydrolysereaktors der Vakuumentwässerungskolonne zuzuführen. Das Sumpfprodukt der Vakuumentwässerungskolonne ist weitgehend wasserfrei und wird der Monoethylenglykolreindestillation zugeführt. Das Kopfprodukt, weitgehend monoethylenglykolfreies Wasser, wird ganz oder teilweise im Prozeß weiterverwendet, insbesondere dem Hydrolysreaktor zugeführt. Der Seitenstrom kann ins Abwasser gegeben oder weiter aufgearbeitet werden.

In einer alternativen Ausführungsform sind zwei Vakuumentwässerungskolonnen hintereinander geschaltet. Der zu reinigende glykolhaltige Strom wird der ersten Vakuumentwässerungskolonne zugeführt. Das Sumpfprodukt der ersten Vakuumentwässerungskolonne wird einer zweiten Vakuumentwässerungskolonne, bevorzugt in deren mittleren Teil, zugeführt. Typische Glykolkonzentrationen im Sumpfprodukt der ersten. Vakuumentwässerungskolonne sind 70-99,5 Gew.-%, bevorzugt 85-99,5 Gew.-%, besonders bevorzugt 95-99 Gew.-%. Am Kopf der zweiten Vakuumentwässerungskolonne wird ein wässriger, weitgehend glykolfreier Strom abgezogen, mit einem Glykolgehalt unter 5 Gew.-%, bevorzugt unter 1 Gew.-%, besonders bevorzugt unter 1000 ppm, in dem Mittelsieder sowie auch Leichtsieder angereichert sind. Das Sumpfprodukt der zweiten Vakuumentwässerungskolonne ist weitgehend wasserfreies Glykol; es wird der Monoethylenglykolreindestillation zugeführt. Die Sumpftemperaturen in der (den) Vakuumentwässerungskolonne(n) sollen in der Regel 220°C nicht überschreiten, bevorzugt werden 120°C bis 200°C, besonders bevorzugt 160°C bis 180°C.

In besonders vorteilhafter Weise wird der einzigen bzw. der letzten Vakuumentwässerungskolonne in deren mittleren Teil ein Kopfstrom aus der Monoethylenglykol-Reindestillation zugeführt. Durch diese Maßnahme können auch Nebenkomponenten, die infolge von Nebenreaktionen in der Monoethylenglykol-Reindestillation entstehen, ausgeschleust werden. Der Kopfstrom ist vorteilhaft gering, inbesondere 1 bis 10 %, bezogen auf den Rein-Monoethylenglykol-Strom. Um den zurückzuführenden Kopfstrom möglichst klein zu halten, müssen die Nebenkomponenten im Kopfstrom aufkonzentriert werden: dazu sind zusätzliche Trennstufen zwischen der Entnahmestelle des Rein-Monoethylenglykols (Seitenabzug) und des zurückzuführenden Stroms notwendig, d.h. in der Monoethylenglykol-Reindestillationskolonne müssen zwischen Kopfabzug und Monoethylenglykolseitenabzug einige Trennstufen realisiert werden, bevorzugt 1 bis 10, besonders bevorzugt 3 bis 6 Trennstufen. Ein vorteilhafter Nebeneffekt der Aufkonzentrierung und Rückführung der Nebenkomponenten ist, daß auch das im Kolonnenfeed zur Monoethylenglykol-Reindestillation in geringen Mengen enthaltene Wasser in die Vakuumentwässerung zurückgeführt wird. Man erhält auf diese Weise ein extrem wasserarmes Monoethylenglykol.

Gemäß einer besonders vorteilhaften Verfahrensvariante werden zusätzlich zur Ausschleusung in der Vakuumentwässerung die Ausschleusung von Nebenkomponenten, insbesondere von Leichtsiedern, in der Druckentwässerungsstufe verbessert. Dazu wird die Druckentwässerungskolonne oder mindestens die erste Druckentwässerungskolonne der Kaskade mit einem Abtriebsteil mit mindestens einer Trennstufe, bevorzugt 2 bis 10 Trennstufen, besonders bevorzugt mit 3 bis 6 Stufen ausgestattet, wobei ein Teil des Kopfstroms der Entwässerungskolonne(n) mit Abtriebsteil ausgeschleust wird.

Herkömmliche großtechnische Verfahren haben den sogenannten Acetaldehydpurge am Sumpfverdampfer der zweiten Druckentwässerungskolonne: hier werden die Brüden der ersten Druckentwässerungskolonne weitgehend kondensiert, der nicht kondensierte Anteil, etwa 1-5 Gew.-% der gesamten Brüden wird ausgeschleust. Die Restbrüden werden ggf. in einem weiteren Wärmeübertrager nachkondensiert, wobei die Kondensationswärme an einer geeigneten Stelle im Gesamtprozeß genutzt werden kann. Nach dieser herkömmlichen Lösung können jedoch nur Nebenkomponenten, die in der ersten Druckentwässerungskolonne als Bestandteil der Brüden abgeführt werden, über den Acetaldehydpurge ausgeschleust werden. Dies ist insbesondere im Fall von Formaldehyd unzureichend, da die Flüchtigkeit von Formaldehyd in wässrigen Glykollösungen mit Zunahme des Glykolgehalts abnimmt, insbesondere infolge von chemischen Reaktionen des Formaldehyds mit Wasser und Glykolen. Um somit Formaldehyd aus dem glykolhaltigen Sumpfprodukt der Druckentwässerungskolonne abzutrennen, ist ein Abtriebsteil in der Druckentwässerungskolonne bzw. mindestens in der ersten Druckentwässerungskolonne einer Kaskade mit mindestens einer Stufe, bevorzugt 2 bis 10 Stufen, besonders bevorzugt 3 bis 6 Stufen, notwendig. Erst wenn der Formaldehyd in die rein wässrigen Brüden der ersten Kolonne abgetrernnt worden ist, kann er zusammen mit Acetaldehyd über den Acetaldehydpurge ausgeschleust werden. Dabei gelingt die Abtrennung des Formaldehyds im Abtriebsteil umso besser, je höher die Temperatur und entsprechend der Druck in der Druckentwässerungskolonne bzw. in der ersten Druckentwässerungskolonne der Kaskade ist und je mehr Wasser im Reaktoraustrag vorhanden ist. Zwei der zusätzlichen Böden im Abtriebsteil können eingespart werden, wenn der Sumpfverdampfer konstruktiv mit "getrenntem Sumpf" entsprechend der DE-C-33 38 488 ausgeführt ist.

Die Menge der ausgeschleusten Nebenkomponenten, inbesondere Acetaldehyd und Formaldehyd, hängt davon ab, wieviel Abwasser ausgeschleust wird. Dabei kann aber die Menge des im Sumpfverdampfer der zweiten Entwässerungskolonne nicht kondensierten Brüdens aus Gründen des Energieverbundes und aus regelungstechnischen Randbedingungen nicht beliebig gesteigert werden. Es wurde eine besonders bevorzugt Verfahrensvariante gefunden, wonach eine weitere Abtrennung von Nebenkomponenten aus dem kondensierten Brüden durch Dampfstrippung möglich ist. Der mit Nebenkomponenten beladene Strippdampf kann anschließend an einer geeigneten Stelle im Prozeß energetisch genutzt werden. Zur Wasserdampfstrippung wird daher keine zusätzliche Energie benötigt, sondern lediglich ein zusätzlicher Apparat. Die Ausschleusung von Nebenkomponenten ist besonders wirkungsvoll. wenn der Ablauf des Strippers als Rücklauf auf die erste Entwässerungskolonne gegeben wird, da durch diese Rückführung der Aldehydgehalt am Kopf der ersten Druckentwässerungskolonne und im Stripper steigt und damit auch die Ausschleusrate zunimmt.

In vorteilhafter Weise beträgt die Temperatur unterhalb der Zulaufstelle zur Druckentwässerungskolonne über 80°C, bevorzugt zwischen 100°C und 250°C, besonders bevorzugt zwischen 115°C und 230°C, wobei der Druck im Abtriebsteil mindestens 1 bar, bevorzugt bei 2 bis 30 bar beträgt.

Vorteilhaft wird der Kopfstrom der Druckentwässerungskolonne(n) mit Abtriebsteil in einen Teilkondensator und/oder einen Stripper, insbesondere einen Wasserdampfstripper, eingeleitet und der (die) mit Nebenkomponenten angereicherte(n) gasförmige Strom (Ströme) ausgeschleust.

In geeigneter Weise werden der Teilkondensator und/oder der Stripper bei Temperaturen über 90°C, bevorzugt zwischen 120 und 250°C, betrieben.

Die Erfindung wird im folgenden anhand einer Zeichnung sowie von Ausführungsbeispielen näher erläutert.

Es zeigen im einzelnen:
- **Figur 1**: ein Schema für ein großtechnisches Verfahren zur Glykolgewinnung nach dem Stand der Technik,
- **Figur 2**: ein Schema eines besonders bevorzugten Verfahrens zur Glykolgewinnung gemäß der Erfindung,
- **Figur 3**: ein Ausführungsbeispiel für ein erfindungsgemäßes Verfahren mit einem Auslaß für Nebenkomponenten als Kopfstrom einer Vakuumentwässerungskolonne und
- **Figur 4**: ein Ausführungsbeispiel für ein erfindungsgemäßes Verfahren mit einer Druckentwässerungskolonne mit Abtriebsteil und einem Auslaß für Nebenkomponenten als Kopfstrom sowie anschließender Konzentrierung in einem Teilkondensator und einem Stripper.

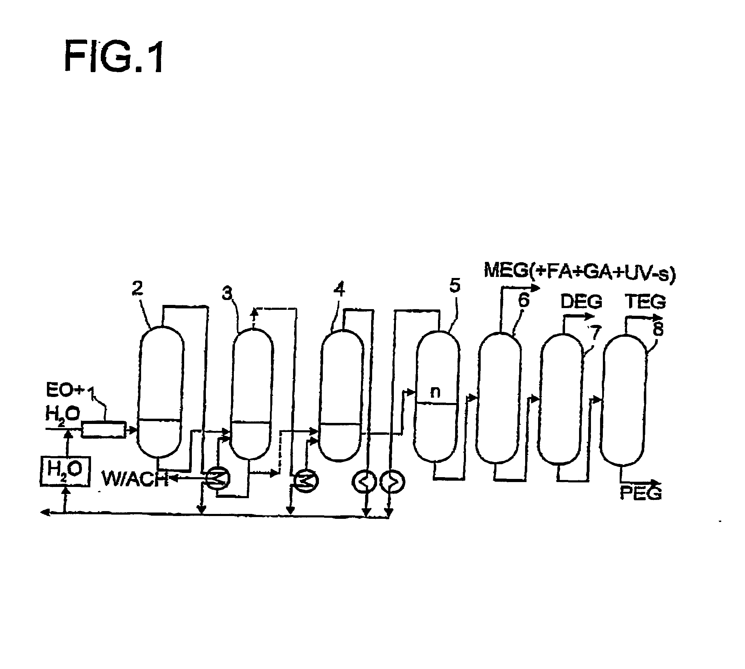

Figur 1 zeigt ein Schema für die großtechnische Gewinnung von Glykol nach dem Stand der Technik. Ein Wasser/Ethylenoxid-Gemisch mit einem Gewichtsverhältnis Wasser:Ethylenoxid von 4:1 bis 15:1 wird dem Hydrolysereaktor 1 zugeführt und anschließend einer Druckentwässerung, die vorliegend als Kaskade mit drei druckgestaffelten Kolonnen 2, 3 und 4 dargestellt ist. Der Zulauf der Kolonnen 2, 3 und 4 liegt jeweils im Sumpf. Der Brüdenstrom der ersten Druckentwässerungskolonne 2 wird im Sumpfverdampfer der zweiten Druckentwässerungskolonne 3 kondensiert und der nicht kondensierte Anteil als sogenannter Acetaldehydpurge (W/ACH, d.h. Wasser/Acetaldehyd) ausgeschleust. Die kondensierten Brüden der Druckentwässerungskolonnen 2, 3 und 4 werden vor den Hydrolysereaktor 1 zurückgeführt. Der Sumpfstrom der letzten Druckentwässerungskolonne 4 wird einer Vakuumentwässerungskolonne 5 in deren mittleren Teil aufgegeben. Der überwiegend wasserhaltige Brüden aus der Vakuumentwässerungskolonne 5 wird ebenfalls kondensiert und vor den Hydrolysereaktor 1 zurückgeführt. Der Sumpfaustrag der Vakuumentwässerungskolonne 5 wird einer Monoethylenglykol-Reindestillationskolonne 6 zugeführt, aus der als Kopfprodukt Monoethylenglykol, sowie Nebenkomponenten, insbesondere Formaldehyd (F_{A}), Glykolaldehyd (GA) und UV-Spoiler (UV-S), abgezogen werden. Der Sumpfaustrag der Monoethylenglykol-Reindestillationskolonne 6 wird einer Diethylenglykol-Reindestillationskolonne 7 zugeführt, aus der als Kopfprodukt reines Diethylenglykol abgezogen und deren Sumpfaustrag einer weiteren Kolonne, der Triethylenglykol-Reindestillationskolonne 8 zugeführt wird. Das Kopfprodukt der Triethylenglykol-Reindestillationskolonne ist reines Triethylenglykol und der Sumpfaustrag der Kolonne 8 enthält eine Mischung höherer Glykole, die als "Polyethylenglykol" (PEG) bezeichnet wird.

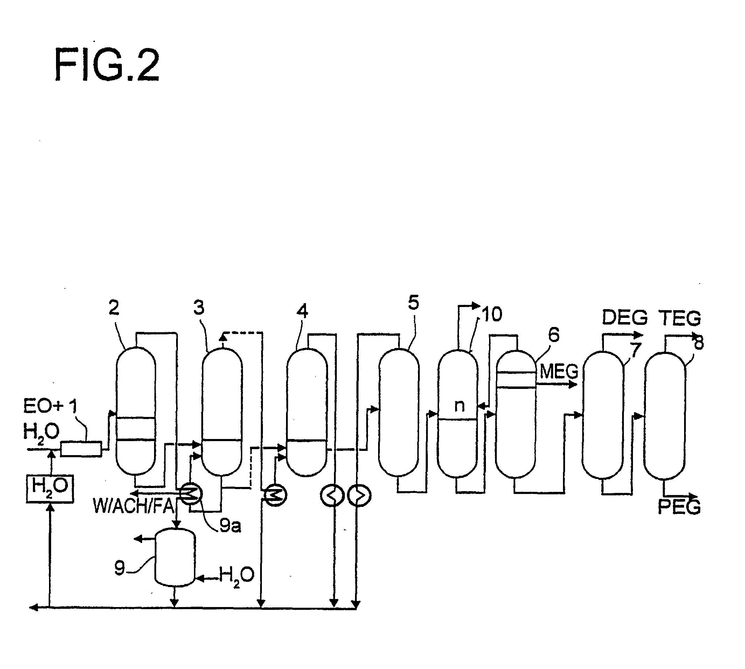

Figur 2 zeigt demgegenüber ein großtechnisches Verfahren zur Gewinnung von hochreinem Monoethylenglykol nach der Erfindung: Gegenüber dem Verfahrensschema in Figur 1 ist der Zulauf zur ersten Druckentwässerungskolonne 2 höher gelegt, und zwar weist diese Druckentwässerungskolonne 2 einen Abtriebsteil mit 2 bis 6 Böden auf.

Ein weiterer Unterschied zum Verfahren nach Figur 1 besteht darin, daß der Brüden der ersten Druckentwässerungskolonne 2 nach Teilkondensation im Sumpfverdampfer der Druckentwässerungskolonne 3 in einem Stripper 9 mit Wasserdampf von Nebenkomponenten freigestrippt wird. Aus dem Stripper wird ein Nebenkomponenten enthaltender gasförmiger Strom (W/ACH/FA, d.h. Wasser/-Acetaldehyd/Formaldehyd) ausgeschleust.

Ein weiterer Unterschied zum Verfahrensschema nach Figur 1 besteht darin, daß am Kopfteil der letzten Vakuumentwässerungskolonne 10 ein Auslaß für einen mit Nebenkomponenten beladenen wässrigen Brüdenstrom geschaffen wird. Weiterhin wird das Hauptprodukt aus der Monoethylenglykol-Reindestillationskolonne 6 nunmehr als Seitenstrom abgezogen und ein Kopfstrom aus der Monoethylenglykol-Reindestillationskolonne 6 in den mittleren Teil der letzten Vakuumentwässerungskolonne 10 zurückgeführt.

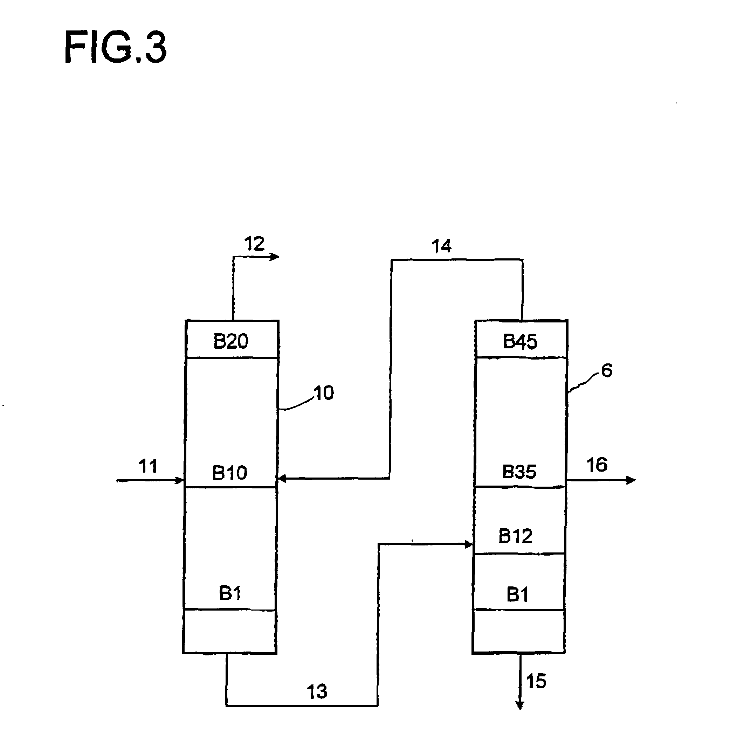

Figur 3 zeigt ein Beispiel für einen erfindungsgemäß geschaffenen Auslaß am Kopf der letzten Vakuumentwässerungskolonne 10. Der Vakuumentwässerungskolonne 10 wird auf den 10. (B10) Boden ein Strom 11 zugeführt und über die Vakuumentwässerungskolonne 10, die mit 20 Glockenböden ausgestattet ist, in einen Kopfstrom 12 und einen Sumpfstrom 13 aufgetrennt. Der Sumpfstrom 13 wird auf den 12. Boden (B12) einer Monoethylenglykol-Reindestillationskolonne 6 mit 45 Glockenböden aufgegeben; über einen Seitenabzug wird vom 35. Boden (B35) ein hochreiner Monoethylenglykolstrom 16 abgezogen. Der Kopfstrom 14 der Monoethylenglykol-Reindestillationskolonne 6 wird auf den 10. Boden (B10) der letzten Vakuumentwässerungskolonne 10 zurückgeführt. Der Sumpfstrom 15 der Monoethylenglykol-Reindestillationskolonne 6 wird den weiteren Reindestillationskolonnen zugeführt. Die Zusammensetzung der Ströme 11-16 ist in der nachfolgenden Tabelle 1 aufgeführt. Daraus ist insbesondere zu erkennen, daß die Konzentration an Nebenkomponenten, insbesondere Acetaldehyd, Formaldehyd und Glykolaldehyd aus dem Zulauf 11 zur letzten Vakuumentwässerungskolonne 10 zum Seitenabzug 16 der Monoethylenglykol-Reindestillationskolonne 6 deutlich abnimmt und gleichzeitig die entsprechende UV-Transmission bei 220 nm sowie bei 275 mm zunimmt.

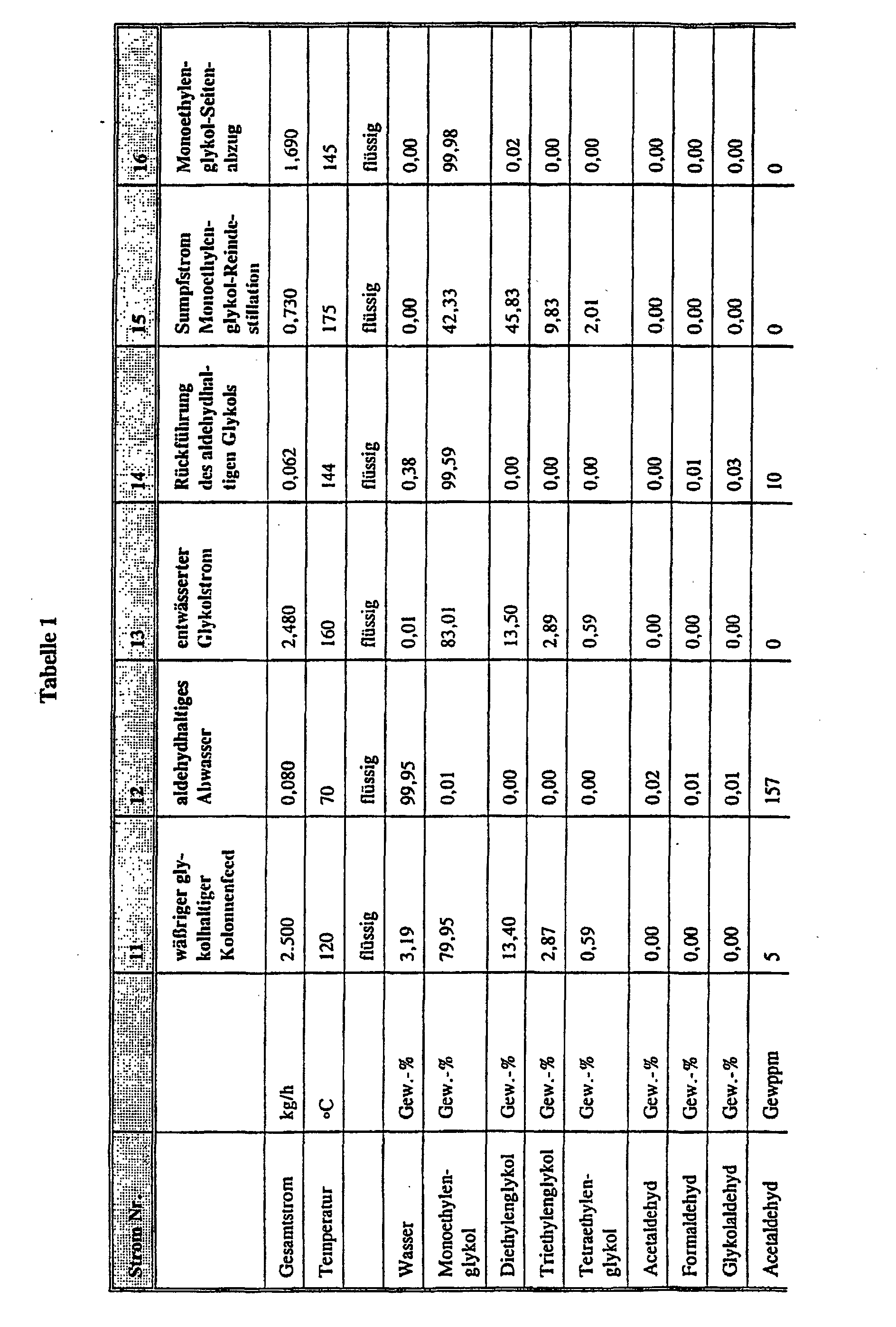

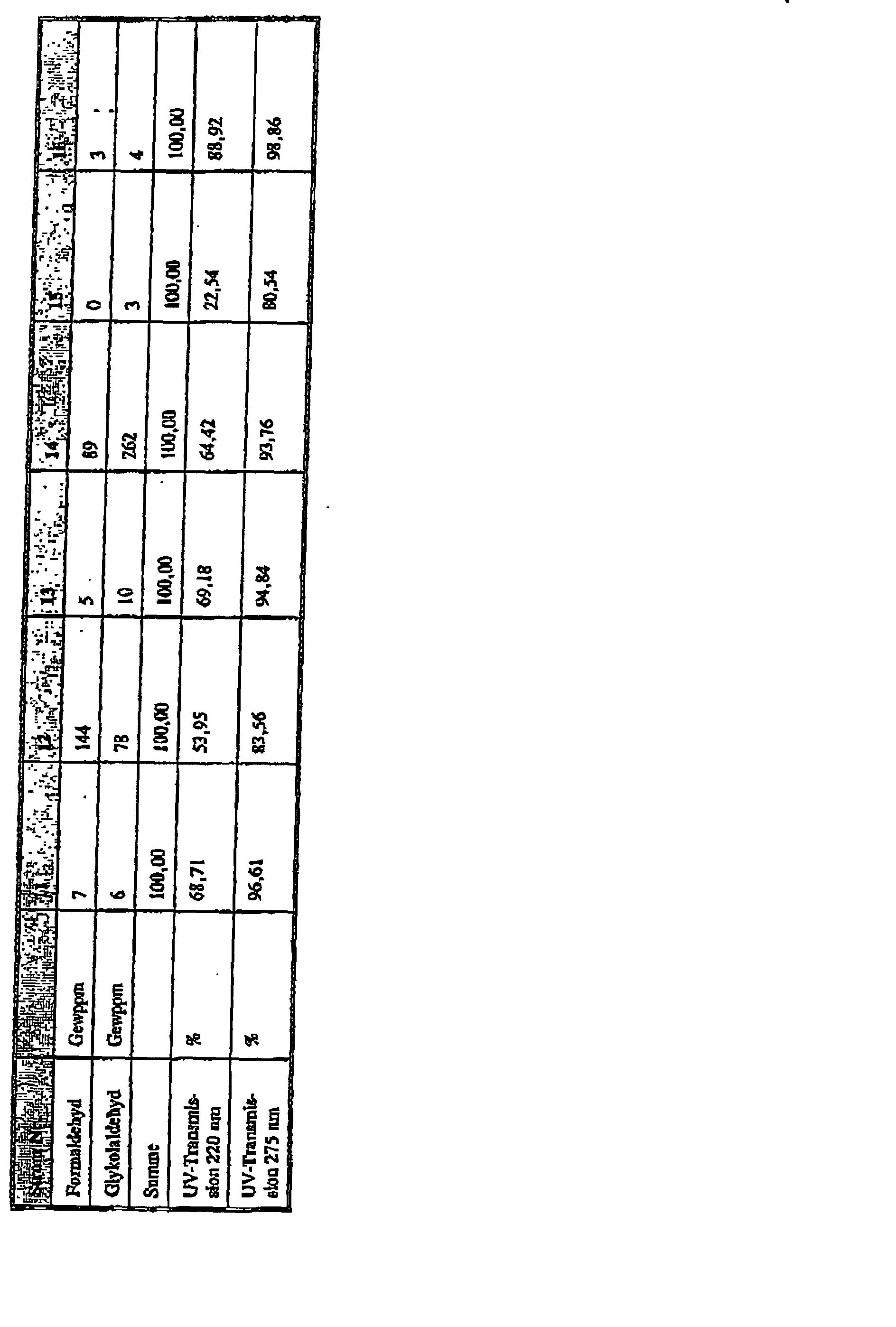

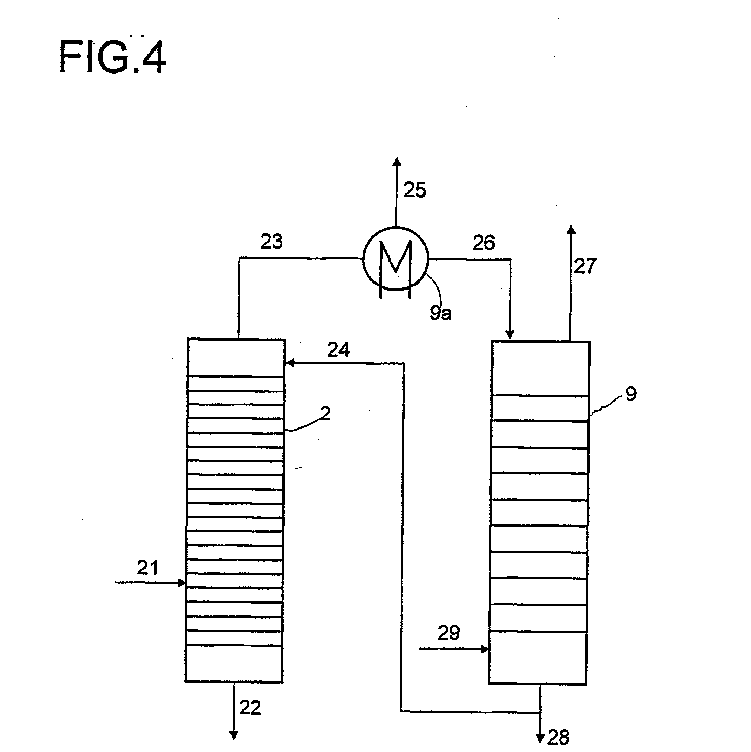

Figur 4 zeigt ein Beispiel für die erfindungsgemäße Ausgestaltung einer Druckentwässerungskolonne 2 mit Abtriebsteil sowie mit einem Stripper 9 zur Aufkonzentrierung der Nebenkomponenten vor deren Ausschleusung. Der Zulauf 21 des aufzutrennenden glykolhaltigen Stroms liegt auf dem 5. Boden einer Druckentwässerungskolonne 2 mit 20 Glockenböden. Deren Kopfstrom 23 wird nach Teilkondensation als Strom 26 auf einen Stripper 9 mit 10 Glockenböden gegeben und im Gegenstrom mit Wasserdampf 29 von Nebenkomponenten freigestrippt. Die Nebenkomponenten enthaltenden gasförmigen Ströme 25 und 27 werden ausgeschleust. Ein Teilstrom des Sumpfaustrags des Strippers 9 bildet als Teilstrom 24 den Rücklauf der Entwässerungskolonne 2. Die Zusammensetzung der Ströme 21-29 ist für ein Verfahren nach der Erfindung in Tabelle 2a aufgeführt. Zum Vergleich ist die Zusammensetzung der Ströme 21-29 für ein Verfahren nach dem Stand der Technik, d.h. mit Druckentwässerungskolonne ohne Abtriebsteil und ohne Stripper in Tabelle 2b aufgeführt.

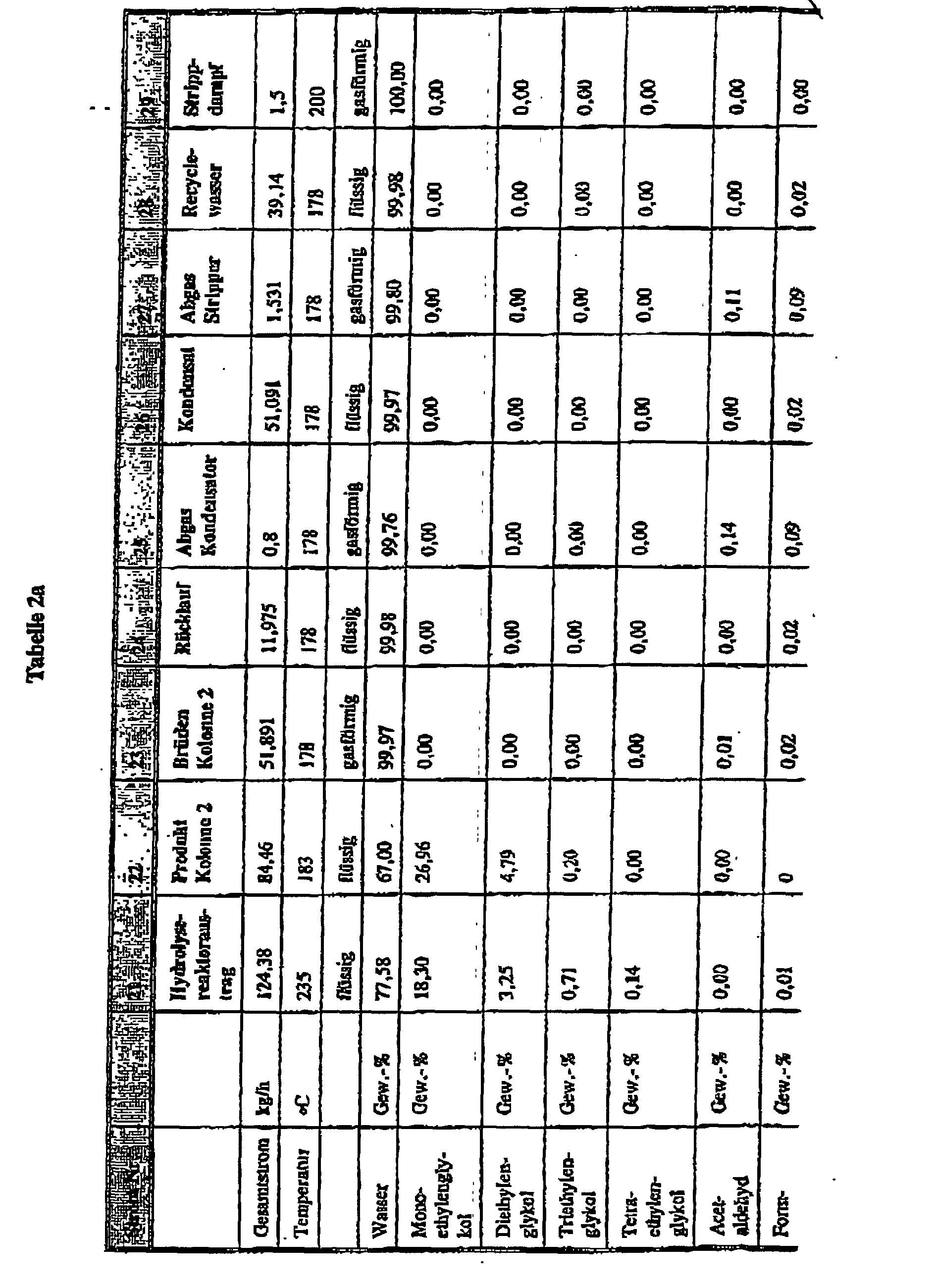

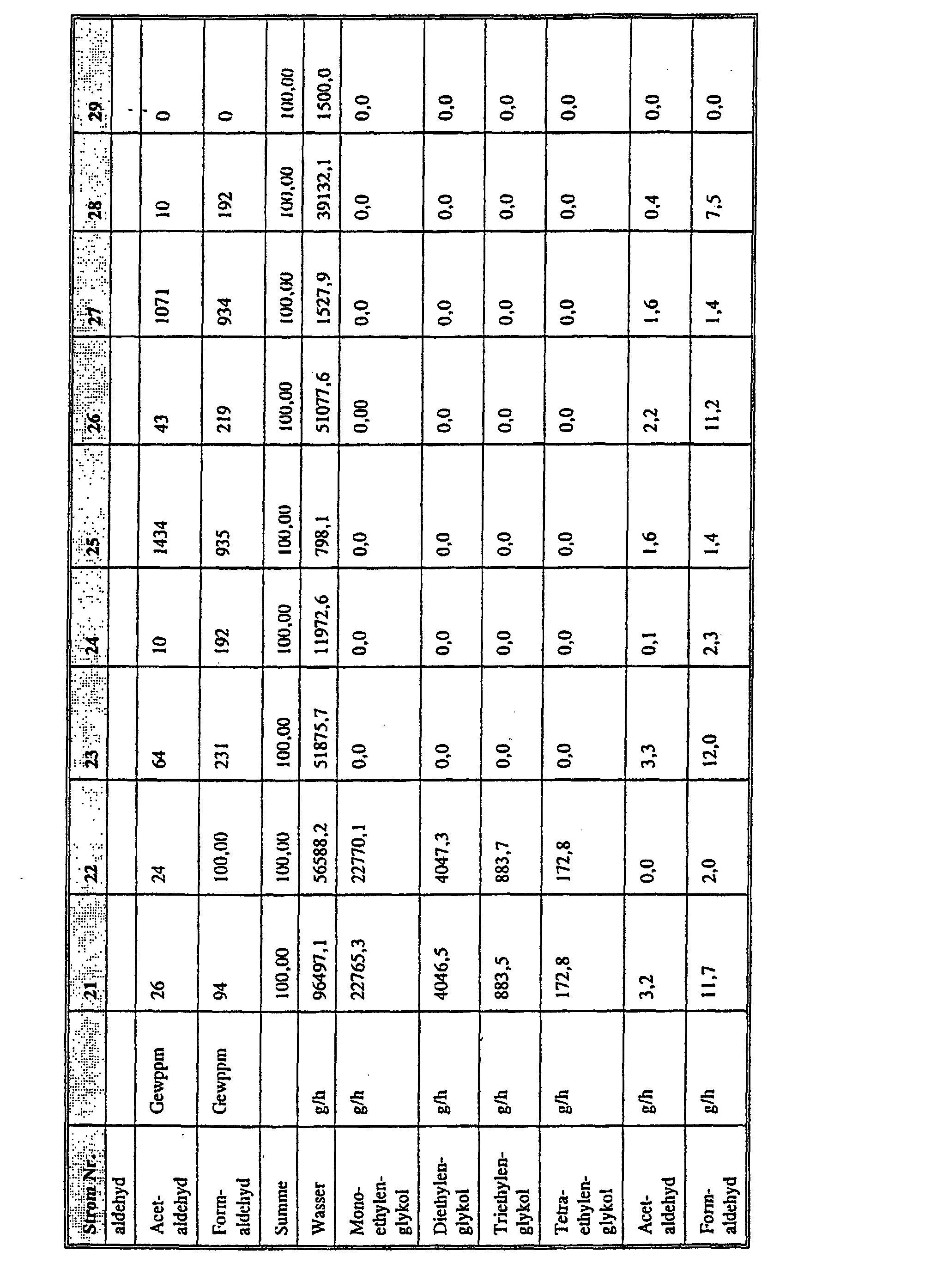

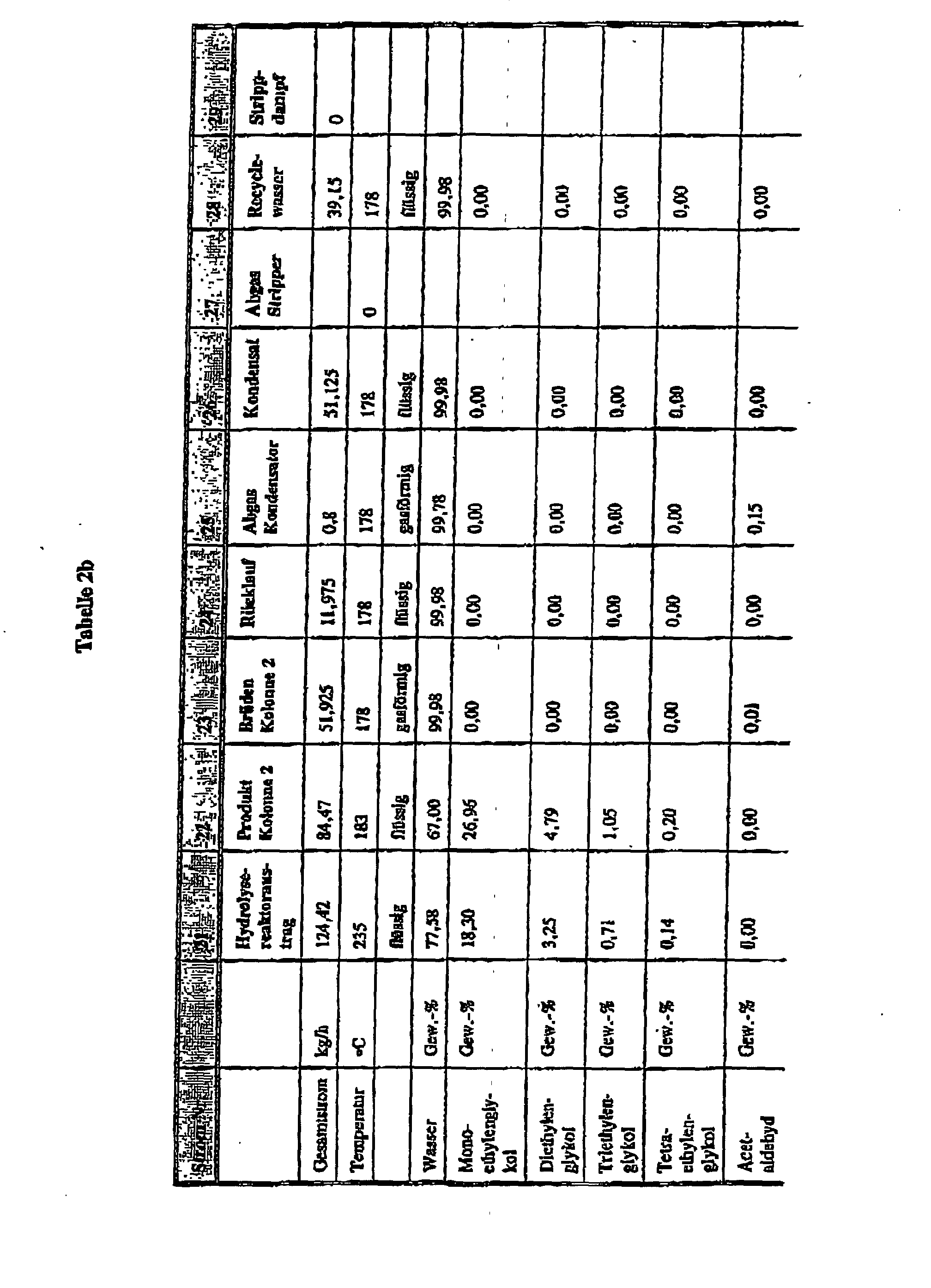

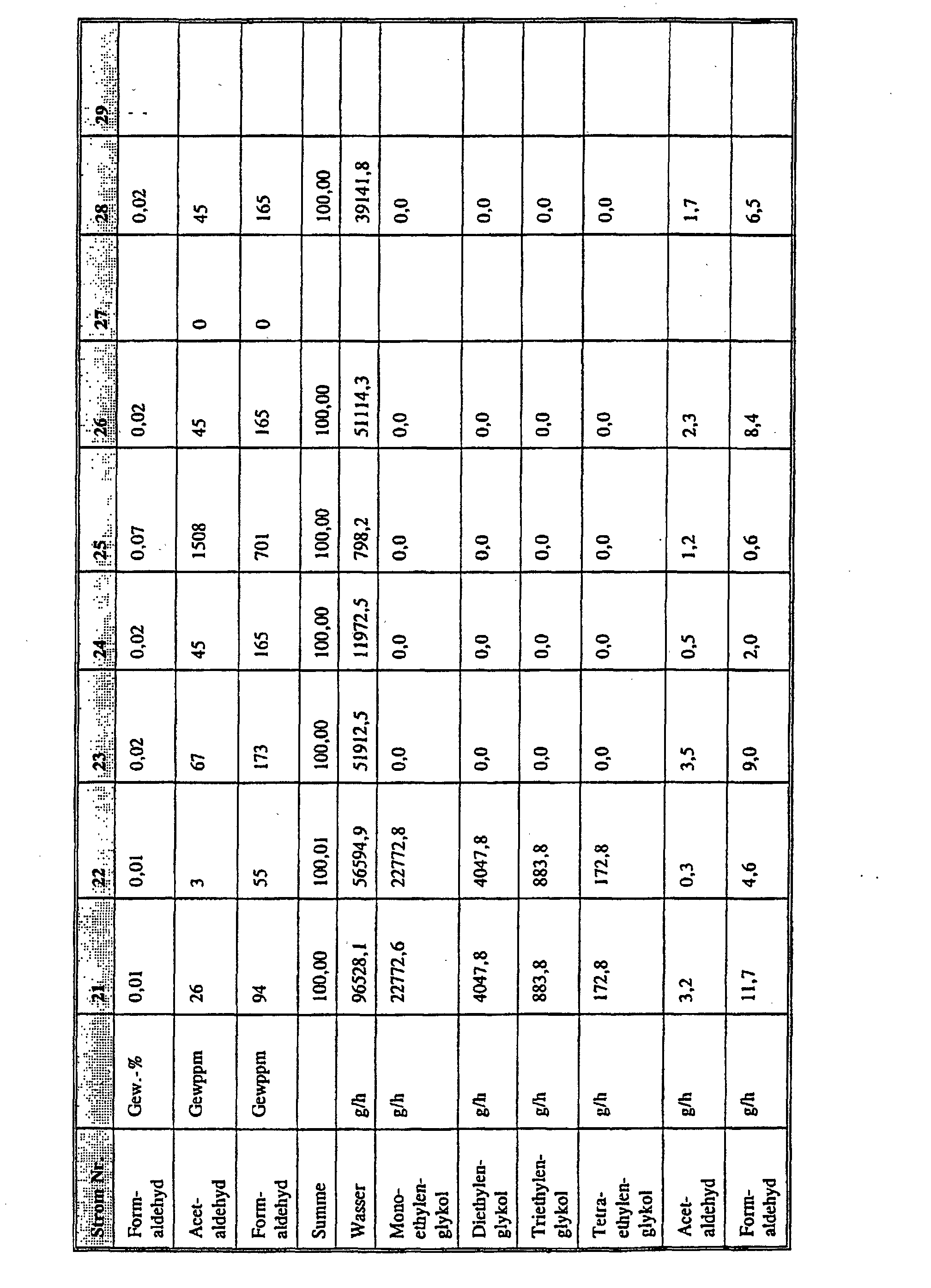

Nach dem erfindungsgemäßen Verfahren wird ein Produktstrom 22 aus der ersten Druckentwässerungskolonne 2 mit weniger Verunreinigungen (0,0 g/h Acetaldehyd und 2,0 g/h Formaldehyd) gegenüber dem Stand der Technik (0,3 g/h Acetaldehyd und 4,6 g/h Formaldehyd) erhalten.

An Nebenkomponenten werden erfindungsgemäß 1,1 g/h Acetaldehyd und 0,7 g/h Formaldehyd in Strom 25 sowie 1,6 g/h Acetaldehyd und 1,4 g/h Formaldehyd in Strom 27 ausgeschleust gegenüber nur 1,2 g/h Acetaldehyd und 0,6 g/h Formaldehyd in Strom 25 nach dem aus dem Stand der Technik bekannten Verfahren.

## Patentansprüche

1. Verfahren zur destillativen Gewinnung von hochreinem Monoethylenglykol aus dem Hydrolyseprodukt von Ethylenoxid durch Druckentwässerung, Vakuumentwässerung und anschließender Reindestillation, wonach bei der Vakuumentwässerung ein wässriger Strom abgezogen wird, der Monoethylenglykol in einer Konzentration unterhalb von 1 Gew.-%, Mittelsieder und Leichtsieder enthält und der, gegebenenfalls nach weiterer Aufarbeitung, ausgeschleust wird, **dadurch gekennzeichnet, dass** die Vakuumentwässerung in einer Vakuumentwässerungs-kolonne (5) stattfindet und daß der wässrige Strom als Seitenstrom aus der Vakuumentwässerungskolonne (5) abgezogen wird, oder daß die Vakuumentwässerung in zwei Vakuumeniwässerungskolonnen (5, 10) stattfindet und daß der wässrige Strom als Kopfstrom der zweiten Vakuumentwässerungskolonne (10) abgezogen wird.

2. Verfahren nach Anspruch 1, **dadurch gekennzeichnet, dass** die Druckentwässerung in einer Kaskade durchgeführt wird.

3. Verfahren nach Anspruch 1 oder 2, **dadurch gekennzeichnet, dass** bei der Vakuumentwässerung ein wäßriger Strom abgezogen wird, der Monoethylenglykol in einer Konzentration unterhalb von 0,1 Gew.-% enthält.

4. Verfahren nach einem der Ansprüche 1 bis 3, **dadurch gekennzeichnet, daß** der Kopfstrom der Monoethylenglykol-Reindestillationskolonne (6) in die letzte Vakuumentwässerungskolonne (10) zurückgeführt wird und das hochreine Monoethylenglykol über einen Seitenabzug der Monoethylenglykol-Reindestillationskolonne (6) abgezogen wird.

5. Verfahren nach Anspruch 4, **dadurch gekennzeichnet, daß** der Kopfstrom der Monoethylenglykol-Reindestillationskolonne (6) 1 bis 10 % des Monoethylenglykol-Seitenabzugstroms beträgt und/oder daß zwischen dem Kopf der Monoethylenglykol-Reindestillationskolonne (6) und dem Seitenabzug 1 bis 10 Trennstufen angeordnet sind.

6. Verfahren nach einem der Ansprüche 1 bis 5, **dadurch gekennzeichnet, daß** die Sumpftemperatur in der (den) Vakuumentwässerungskolonne(n) (5, 10) 220°C nicht übetschreitet.

7. Verfahren nach Anspruch 6, **dadurch gekennzeichnet, dass** die Sumpftemperatur in der (den) Vakuumentwässerungskolonne(n) (5, 10) zwischen 160°C und 180°C liegt.

8. Verfahren nach einem der Ansprüche 1 bis 7, **dadurch gekennzeichnet, daß** die Druckentwässerungskolonne (2) oder mindestens die erste Druckentwässerungskolonne (2) der Kaskade (2, 3, 4) einen Abtriebsteil mit mindestens einer Trennstufe aufweist und daß ein Teil des Kopfstroms der Druckentwässerungskolonne(n) mit Abtriebsteil ausgeschleust wird.

9. Verfahren nach Anspruch 8, **dadurch gekennzeichnet, daß** die Temperatur unterhalb der Zulaufstelle zur Druckentwässerungskolonne (2) über 80°C und daß der Druck im Abtriebsteil bei mindestens 1 bar liegt.

10. Verfahren nach Anspruch 8 oder 9, **dadurch gekennzeichnet, daß** der Kopfstrom der Druckentwässerungskolonne(n) (2) mit Abtriebsteil in einen Teilkondensator (9a) und/oder einen Stripper (9) eingeleitet wird und daß der (die) an der Nebenkomponenten angereicherte(n) gasförmige Strom (Ströme) ausgeschleust wird.

11. Verfahren nach Anspruch 10, **dadurch gekennzeichnet, daß** Teilkondensator (9a) und Stripper (9) bei Temperaturen über 90°C betrieben werden.

## Claims

1. A process for the distillative recovery of high purity monoethylene glycol from the hydrolysis product of ethylene oxide by pressure dewatering, vacuum dewatering and subsequent purifying distillation, which comprises withdrawing during the vacuum dewatering an aqueous stream which contains monoethylene glycol in a concentration below 1% by weight, medium boilers and low boilers and which, optionally after further workup, is removed from the system, **characterized in that** the vacuum dewatering takes place in a vacuum dewatering column (5) and **in that** the aqueous stream is withdrawn as a sidestream from the vacuum dewatering column (5), or **in that** the vacuum dewatering takes place in two vacuum dewatering columns (5, 10) and **in that** the aqueous stream is withdrawn as overhead stream of the second vacuum dewatering column (10).

2. A process as claimed in claim 1, **characterized in that** the pressure dewatering is carried out in a battery.

3. A process as claimed in claim 1 or 2, **characterized in that** the aqueous stream withdrawn in the course of the vacuum dewatering contains monoethylene glycol in a concentration below 0.1% by weight.

4. A process as claimed in any one of claims 1 to 3, **characterized in that** the overhead stream of the monoethylene glycol purifying distillation column (6) is returned into the last vacuum dewatering column (10) and the high purity monoethylene glycol is withdrawn via a side takeoff of the monoethylene glycol purifying distillation column (6).

5. A process as claimed in claim 4, **characterized in that** the overhead stream of the monoethylene glycol purifying distillation column (6) amounts to from 1 to 10% of the monoethylene glycol side takeoff stream and/or **in that** from 1 to 10 separating stages are disposed between the top of the monoethylene glycol purifying distillation column (6) and the side takeoff.

6. A process as claimed in any one of claims 1 to 5, **characterized in that** the base-of-column temperature in the vacuum dewatering column(s) (5, 10) does not exceed 220°C.

7. A process as claimed in claim 6, **characterized in that** the base-of-column temperature in the vacuum dewatering column(s) (5, 10) is between 160°C and 180°C.

8. A process as claimed in any one of claims 1 to 7, **characterized in that** the pressure dewatering column (2) or at least the first pressure dewatering column (2) of the battery (2, 3, 4) has a stripping section with at least one separating stage and **in that** a portion of the overhead stream of the pressure dewatering column(s) having a stripping section is removed from the system.

9. A process as claimed in claim 8, **characterized in that** the temperature below the feed point into the pressure dewatering column (2) is above 80°C and **in that** the pressure in the stripping section is not less than 1 bar.

10. A process as claimed in claim 8 or 9, **characterized in that** the overhead stream of the pressure dewatering column(s) (2) having a stripping section is introduced into a partial condenser (9a) and/or a stripper (9) and **in that** the gaseous streams enriched with the secondary component is(are) removed from the system.

11. A process as claimed in claim 10, **characterized in that** the partial condenser (9a) and the stripper (9) are operated at above 90°C.

## Revendications

1. Procédé de production par distillation de monoéthylèneglycol de haute pureté à partir du produit d'hydrolyse de l'oxyde d'éthylène au moyen d'une déshydratation sous pression, d'une déshydratation sous vide puis d'une rectification, selon lequel, lors de la déshydratation sous vide, on soutire un courant aqueux qui contient du monoéthylèneglycol à une concentration inférieure à 1% en poids, des produits à point d'ébullition intermédiaire et à bas point d'ébullition, et qui est éventuellement soutiré après un retraitement supplémentaire, **caractérisé en ce que** la déshydratation sous vide se déroule dans une colonne de déshydratation sous vide (5) et que le courant aqueux est soutiré de la colonne de déshydratation sous vide (5) en tant que courant latéral, ou **en ce que** la déshydratation sous vide se déroule dans deux colonnes de déshydratation sous vide (5, 10) et que le courant aqueux est soutiré de la seconde colonne de déshydratation sous vide (10) en tant que courant de tête.

2. Procédé selon la revendication 1, **caractérisé en ce que** la déshydratation sous pression est réalisée en cascade.

3. Procédé selon la revendication 1 ou 2, **caractérisé en ce que** l'on soutire, lors de la déshydratation sous vide, un courant aqueux qui contient du monoéthylèneglycol à une concentration inférieure à 0,1% en poids.

4. Procédé selon l'une quelconque des revendications 1 à 3, **caractérisé en ce que** le courant de tête de la colonne de rectification du monoéthylèneglycol (6) est recyclé dans la dernière colonne de déshydratation sous vide (10) et que le monoéthylèneglycol de haute pureté est soutiré de la colonne de rectification du monoéthylèneglycol (6) par l'intermédiaire d'une canalisation latérale.

5. Procédé selon la revendication 4, **caractérisé en ce que** le courant de tête de la colonne de rectification du monoéthylèneglycol (6) représente 1% à 10% du courant de la canalisation latérale du monoéthylèneglycol et/ou que 1 à 10 étages de séparation sont disposés entre la tête de la colonne de rectification du monoéthylèneglycol (6) et la canalisation latérale.

6. Procédé selon l'une quelconque des revendications 1 à 5, **caractérisé en ce que** la température de fond dans la ou les colonnes de déshydratation sous vide (5, 10) ne dépasse pas 220°C.

7. Procédé selon la revendication 6, **caractérisé en ce que** la température de fond dans la ou les colonnes de déshydratation sous vide (5, 10) se situe entre 160°C et 180°C.

8. Procédé selon l'une quelconque des revendications 1 à 7, **caractérisé en ce que** la colonne de déshydratation sous pression (2) ou au moins la première colonne de déshydratation sous pression (2) de la cascade (2, 3, 4) présente une zone de rectification comprenant au moins un étage de séparation et qu'une partie du courant de tête de la ou des colonnes de déshydratation sous pression présentant une zone de rectification est soutiré.

9. Procédé selon la revendication 8, **caractérisé en ce que** la température au-dessous de la zone d'alimentation de la colonne de déshydratation sous vide (2) est supérieure à 80°C et que la pression dans la zone de rectification est d'au moins 1 bar.

10. Procédé selon la revendication 8 ou 9, **caractérisé en ce que** le courant de tête de la colonne de déshydratation sous vide (2) présentant une zone de rectification est introduit dans un condenseur partiel (9a) et/ou dans un revaporisateur (9) et que le ou les courants gazeux enrichis en sous-composants sont soutirés.

11. Procédé selon la revendication 10, **caractérisé en ce que** le condenseur partiel (9a) et le revaporisateur (9) fonctionnent à des températures supérieures à 90°C.
